# EUROPEAN PATENT APPLICATION

(11) **EP 2 123 642 A1**
(43) Date of publication of application: **25.11.2009**
(21) Application number: 08722105.7
(22) Date of filing: 14.03.2008
(51) Int. Cl.: C07D 265/30, A61K 31/5375, A61P 3/10, A61P 25/04

(54) **NOVEL PROPHYLACTIC AND/OR THERAPEUTIC AGENT FOR NEUROGENIC PAIN**

(30) Priority: 15.03.2007 JP 2007066727
(71) Applicant: Astellas Pharma Inc., Tokyo 103-8411 (JP)
(72) Inventor: AOKI, Toshiaki, Tokyo 103-8411 (JP); TAMURA, Seiji, Tokyo 103-8411 (JP)
(74) Representative: Albrecht, Thomas
(86) International application number: PCT/JP2008/054709
(87) International publication number: WO 2008/111668

(57) **Abstract**

The present invention relates to an agent for preventing and/or treating neuropathic pain comprising a 2-[(substituted-inden-7-yloxy)methyl]morpholine of the formula (I) or a pharmaceutically acceptable salt thereof as an active ingredient. The invention is useful for providing an excellent agent for preventing and/or treating neuropathic pain, and particularly useful for providing an agent for preventing and/or treating allodynia, hyperalgesia, hyperesthesia, spontaneous pain, cancer pain, trigeminal neuralgia, phantom Limb pain, postherpetic neuralgia, fibromyalgia, low back/leg pain, thalamic pain, entrapment (compressive) peripheral neuropathy, atypical facial pain, pain accompanying spinal cord injury, pain accompanying multiple sclerosis, pain accompanying chemotherapy-induced neuropathy, cancer pain on which an analgesic effect of a narcotic analgesic such as morphine is insufficient, pain accompanying diabetic neuropathy, and the like.

## Description

### Technical Field

The present invention relates to a novel pharmaceutical use of a morpholine derivative or a pharmaceutically acceptable salt thereof as a preventive and/or therapeutic agent for neuropathic pain.

### Background Art

Neuropathic pain is the intractable pain caused by dysfunction in the peripheral or central nervous system. Examples of the pain condition include pain accompanying diabetic neuropathy, postherpetic neuralgia, fibromyalgia, low back/leg pain, and thalamic pain as typical diseases. Although the onset mechanism of neuropathic pain has many unknown aspects, abnormal tonic discharge of sensory nerves is thought to be the causes. Typical symptoms of neuropathic pain include spontaneous pain, and also allodynia, hyperalgesia, hyperesthesia, and the like. In these symptoms, characteristic pain expressed as "burning", "stringing", "electric shock-like", or the like is manifested. It is often difficult to treat neuropathic pain with analgesics which are effective in common nociceptive pain, such as narcotic analgesics, antiinflammatory analgesics (The Lancet, vol. 353, pp. 1959-1966, 1999). For example, it is known that morphine has a potent analgesic effect on acute nociceptive pain, but does not exhibit a sufficient effect on neuropathic pain. Further, the insufficient analgesic effect of morphine is a major characteristic of neuropathic pain, therefore, it is also used for one of diagnosis of neuropathic pain (Igaku no Ayumi (Journal of Clinical and Experimental Medicine), vol. 189, No. 10, pp. 751-755, 1999). The reason why morphine has almost no effect on neuropathic pain is believed to be due to a functional or morphological change in the nerve caused by neuropathy, that is, dysfunction of inhibitory neurons or a decreased density of opioid receptors in neuropathic state(Saishin Nou to Shinkeikagaku Series, vol. 6, Itami no Shinkeikagaku, Medical View, p. 97, 1997). Further, it is also known that a nonsteroidal antiinflammatory analgesic has almost no effect on neuropathic pain (The Lancet, vol. 353, pp. 1959-1966, 1999, and Igaku no Ayumi (Journal of Clinical and Experimental Medicine), vol. 203, No. 1, pp. 65-69, 2002). This is attributable to the fact that the onset of pathogenic conditions of neuropathic pain is different from that of inflammatory pain. That is, inflammatory pain is induced by the activation of sensory receptors (nociceptors) in normal somatosensory nerve. The activation is caused by noxious stimulus such as inflammatory chemical mediators released after tissue injury, disease, or inflammation (The Clinical Journal of Pain, vol. 16, pp. S131-S138, 2000). Antiinflammatory analgesics exhibit an analgesic effect by suppressing the production of inflammatory chemical mediators. On the other hand, neuropathic pain is defined by IASP as "pain initiated or caused by a primary lesion or dysfunction in the nervous system" (Classification of Chronic Pain, International Association for the Study of Pain (IASP) Task Force on Taxonomy IASP Press: Seattle, pp. 209-214, 1994). A certain type of neuropathic pain is caused by lesion or dysfunction in the peripheral nervous system. Due to the lesion, the expression of important transducer molecules, transmitters, and ion channels are changed, and excitability of peripheral neurons is changed. One of the characteristics of symptoms is an allodynia symptom in which pain is usually caused by a non-noxious stimulus and hyperalgesia in which a noxious stimulus is abnormally strongly amplified (The Clinical Journal of Pain, vol. 16, pp. S131-S138, 2000).

As a method for treating neuropathic pain, neurosurgical interventions such as nerve block or spinal epidural electric stimulation (Igaku no Ayumi (Journal of Clinical and Experimental Medicine), vol. 189, No. 10, pp. 757-762, 1999), pharmacological interventions such as antidepressants (Rinsho to Yakubutsu Chiryo (Clinics and Drug Therapy), vol. 18, No. 7, pp. 643-646, 1999), antiepileptic agents (Clinical Theraputics, vol. 25, pp. 2506-2538, 2003), or the like is employed. However, satisfactory treatment methods in terms of the efficacy and safety have not been established, and the development of more efficacious therapeutic agent in neuropathic pain has been desired.

As antidepressants used for the treatment of neuropathic pain, a certain type of tricyclic antidepressant such as 5-(3-dimethylaminopropylidene)dibenzo[a,d][1,4]-cycloheptadiene (amitriptyline) has been used (Non-patent document 1). It is known that a certain type of tricyclic antidepressant such as amitriptyline has a serotonin and norepinephrine reuptake inhibitory activity and exhibits an analgesic activity through a descending pain inhibitory pathway by increasing the concentrations of the monoamines in synapses (Rinsho to Yakubutsu Chiryo (Clinics and Drug Therapy), vol. 18, No. 7, pp. 643-646, 1999). Amitriptyline which inhibits reuptake of both serotonin and norepinephrine has a superior analgesic effect to 10,11-dihydro-N-methyl-5H-dibenzo[b,f]azepine-5-propanamine (desipramine) which is a tricyclic antidepressant selectively inhibiting reuptake of norepinephrine. Therefore, it is suggested that inhibition of reuptake of both serotonin and norepinephrine is important for an analgesic activity (International Association of the Study of Pain, vol. 21, pp. 169-183, 2001).

Further, recently, it has been reported that a selective serotonin and norepinephrine reuptake inhibitor (SNRI) such as (S)-N-methyl-y-(1-naphthalenyloxy)-2-thiophenepropanamine (duloxetine) or (±)-1-[2-(dimethylamino)-1-(4-methoxyphenyl)ethyl]cyclohexanol (venlafaxine) in which the side effect profiles of the tricyclic antidepressants have been improved shows an efficacy in patients with neuropathic pain (Non-patent document 2). Cis-(±)-2-(aminomethyl)-N,N-diethyl-1-phenylcyclopropanecarboxamide (milnacipran) is also known as SNRI.

On the other hand, (±)-2-[(inden-7-yloxy)methyl]morpholine hydrochloride (indeloxazine hydrochloride) had been used for the treatment of psychiatric symptoms in patients with cerebrovascular disorder in Japan and South Korea (Patent document 1 and Non-patent document 3). In rat studies, it has a high affinity for a serotonin and norepinephrine uptake site and is known to have a serotonin and norepinephrine reuptake inhibitory activity in brain and an antidepressive activity. Further, its optically active substances, (+)-2-[(inden-7-yloxy)methyl]morpholine hydrochloride and (-)-2-[(inden-7-yloxy)methyl]morpholine hydrochloride are also known to exhibit a serotonin and norepinephrine reuptake inhibitory activity in the same manner as (±)-2-[(inden-7-yloxy)methyl]morpholine hydrochloride (Non-patent document 4). Further, it is known that (S)-2-{[(7-fluoroindan-4-yl) oxy]methyl}morpholine hydrochloride has an activity of enhancing neurotransmission by norepinephrine because of having both of an activity of enhancing neurotransmission by serotonin based on a serotonin reuptake inhibitory activity and 5-HT2A receptor antagonism (Non-patent document 5 and Non-patent document 6), and is useful as a therapeutic agent for anxiety or depression and an agent for accelerating recovery from functional disability after onset of central nervous system disease (Patent documents 2 and 3).

Venlafaxine, milnacipran, duloxetine, amitriptyline and the like are known to be effective in rat models of neuropathic pain (Non-patent document 7 and Non-patent document 8). However, it has not been reported that a morpholine derivative including indeloxazine is effective in neuropathic pain.

Patent document 1: US Patent No. 4109088
Patent document 2: US Patent No. 5521180
Patent document 3: US Patent Application Publication No. 2007/0259865
Non-patent document 1: Neurology, vol. 38, pp. 1427-1432, 1988
Non-patent document 2: Human Psycopharmacology, vol. 19, pp. S21-S25, 2004
Non-patent document 3: Neuropharmacology, vol. 37, pp. 1169-1176, 1998
Non-patent document 4: Chemical and Pharmaceutical Bulletin, vol. 33, No. 9, pp. 3766-3774, 1985
Non-patent document 5: European Journal of Pharmacology, vol. 395, No. 1, pp. 31-36, 2000
Non-patent document 6: The Journal of Pharmacology and Experimental Therapeutics, vol. 302, No. 3, pp. 983-991, 2002
Non-patent document 7: The Journal of Pharmacology and Experimental Therapeutics, vol. 311, No. 2, pp. 576-584, 2004
Non-patent document 8: Neuropharmacology, vol. 48, pp. 252-263, 2005

### Disclosure of the invention

### Problems to be solved by the invention

An object of the present invention is to provide a novel agent for preventing and/or treating neuropathic pain compared with conventional SNRI.

### Means for Solving the Problems

The present inventors studies based on their own conception for achieving the above object and found that a morpholine derivative of the present invention exhibits a remarkable therapeutic effect on neuropathic pain compared with conventional SNRI, and thus, the invention has been completed.

An object of the invention is to provide an agent for preventing and/or treating neuropathic pain comprising the morpholine derivative or a pharmaceutically acceptable salt thereof as an active ingredient.
Another object of the invention is to provide a pharmaceutical composition comprising an effective amount of the morpholine derivative or a pharmaceutically acceptable salt thereof and a pharmaceutically acceptable carrier for preventing and/or treating neuropathic pain.
Still another object of the invention is to provide use of the morpholine derivative or a pharmaceutically acceptable salt thereof for manufacture of a medicament for preventing and/or treating neuropathic pain.
Still another object of the invention is to provide a method for preventing and/or treating neuropathic pain comprising administering an effective amount of the morpholine derivative or a pharmaceutically acceptable salt thereof.
Still another object of the invention is to provide a method for producing a pharmaceutical composition for preventing and/or treating neuropathic pain comprising mixing the morpholine derivative or a pharmaceutically acceptable salt thereof with a pharmaceutically acceptable excipient.
Still another object of the invention is to provide a commercial package including a pharmaceutical composition containing the morpholine derivative or a pharmaceutically acceptable salt thereof as an active ingredient, and a description that the morpholine derivative or a pharmaceutically acceptable salt thereof can be used or should be used for preventing and/or treating neuropathic pain.

The invention relates to an agent for preventing and/or treating neuropathic pain comprising a morpholine derivative represented by the formula (I) or a pharmaceutically acceptable salt thereof as an active ingredient. 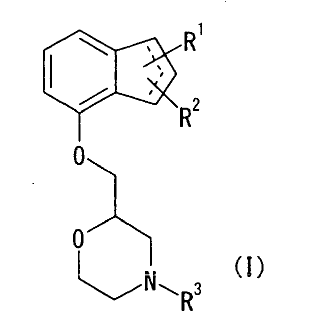 R¹ and R² are the same or different and represent a hydrogen atom, a lower alkyl , or a phenyl ;
R³ represents a hydrogen, a lower alkyl , a phenyl , or a benzyl ; and
a dotted line indicates that a double bond can be formed.

Further, the invention relates to an agent for preventing and/or treating neuropathic pain comprising (±)-2-[(inden-7-yloxy)methyl]morpholine or a pharmaceutically acceptable salt thereof as an active ingredient.

Further, the invention relates to an agent for preventing and/or treating neuropathic pain comprising (+)-2-[(inden-7-yloxy)methyl]morpholine or a pharmaceutically acceptable salt thereof as an active ingredient.

Further, the invention relates to an agent for preventing and/or treating neuropathic pain comprising (-)-2-[(inden-7-yloxy)methyl]morpholine or a pharmaceutically acceptable salt thereof as an active ingredient.

### Effect of the Invention

The invention is useful for providing an excellent preventive and/or therapeutic agent for neuropathic pain. Further, the invention is particularly useful for providing a preventive and/or therapeutic agent for allodynia, hyperalgesia, hyperesthesia, spontaneous pain, cancer pain, trigeminal neuralgia, phantom limb pain, postherpetic neuralgia, fibromyalgia, low back/leg pain, thalamic pain, entrapment (compressive) peripheral neuropathy, pain accompanying central neuropathy, atypical facial pain, pain accompanying spinal cord injury, pain accompanying multiple sclerosis, pain accompanying chemotherapy-induced neuropathy, cancer pain on which an analgesic effect of a narcotic analgesic such as morphine is insufficient, pain accompanying diabetic neuropathy, and the like.

### Best Mode for Carrying Out the Invention

Hereinafter, preferred embodiments of the invention is described.
(1) An agent for preventing and/or treating allodynia, hyperalgesia, hyperesthesia, spontaneous pain, cancer pain, trigeminal neuralgia, phantom limb pain, postherpetic neuralgia, fibromyalgia, low back/leg pain, thalamic pain, entrapment (compressive) peripheral neuropathy, atypical facial pain, pain accompanying spinal cord injury, pain accompanying multiple sclerosis, pain accompanying chemotherapy-induced neuropathy, cancer pain on which an analgesic effect of a narcotic analgesic such as morphine is insufficient, or pain accompanying diabetic neuropathy comprising the morpholine derivative represented by the formula (I) or a pharmaceutically acceptable salt thereof as an active ingredient.
(2) An agent for preventing and/or treating pain accompanying diabetic neuropathy comprising the morpholine derivative represented by the formula (I) or a pharmaceutically acceptable salt thereof as an active ingredient.

(3) The agent described in (1) wherein the compound represented by the formula (I) is (±)-2-[(inden-7-yloxy)methyl]morpholine or a pharmaceutically acceptable salt thereof.
(4) The agent described in (2) wherein the compound represented by the formula (I) is (±)-2-[(inden-7-yloxy)methyl]morpholine or a pharmaceutically acceptable salt thereof.

(5) The agent described in (1) wherein the compound represented by the formula (I) is (+)-2-[(inden-7-yloxy)methyl]morpholine or a pharmaceutically acceptable salt thereof.
(6) The agent described in (2) wherein the compound represented by the formula (I) is (+)-2-[(inden-7-yloxy)methyl]morpholine or a pharmaceutically acceptable salt thereof.

(7) The agent described in (1) wherein the compound represented by the formula (I) is (-)-2-[(inden-7-yloxy)methyl]morpholine or a pharmaceutically acceptable salt thereof.
(8) The agent described in (2) wherein the compound represented by the formula (I) is (-)-2-[(inden-7-yloxy)methyl]morpholine or a pharmaceutically acceptable salt thereof.

(9) A preferred compound of the morpholine derivative represented by the formula (I): 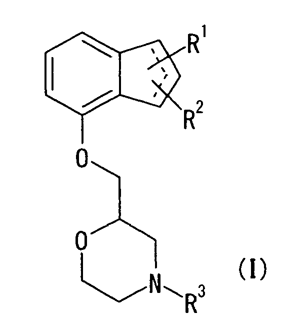 (wherein R¹, R², and R³ have the same meanings as defined above, respectively) is a compound of the formula (II): 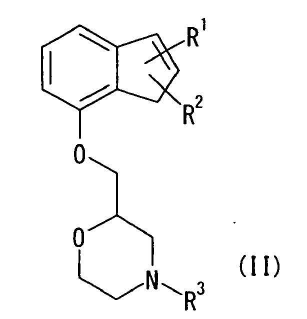 or the formula (III): 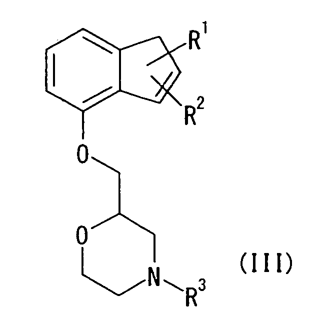 (wherein R¹, R², and R³ have the same meanings as defined above, respectively). More preferred is a compound of the formula (II) (wherein R¹, R², and R³ have the same meanings as defined above, respectively), and further more preferred is a compound of the formula (II) (wherein R¹, R², and R³ are all hydrogen), i.e., (±)-2-[(inden-7-yloxy)methyl]morpholine.

As the pharmaceutically acceptable salt of the morpholine derivative represented by the formula (I) or a pharmaceutically acceptable salt thereof, a hydrochloride is preferred.

(+)-2-[(inden-7-yloxy)methyl]morpholine or a pharmaceutically acceptable salt thereof has an extremely weak CYP inhibitory activity, therefore, the compounds are particularly excellent, for example, when it is used for a patient who takes another medicinal agent, because it can be safely administered with little concern of drug interaction.

In the description mentioned above or below of this specification, preferred examples of various definitions included in the scope of the invention are described in detail hereunder.

The "lower alkyl" means a linear or branched aliphatic hydrocarbon having 1 to 6 carbon atoms. Examples thereof include methyl, ethyl, propyl, isopropyl, butyl, isobutyl, tert-butyl, pentyl, and hexyl and the like.

The "neuropathic pain" means that an abnormal perception of pain sensation including a reduction of pain threshold and the like is continued due to the functional abnormalities brought about neuropathy in which a nerve, plexus or perineural soft tissue are damaged or degenerated. These abnormalities in neuropathy are caused by wound, compression, infection, cancer, ischemia, and the like, or metabolic disorders such as diabetes and the like. Specifically, the neuropathic pain includes unbearable spontaneous pain, allodynia (pain sensation induced by a non-noxious mechanical or thermal stimulus), hyperalgesia (an excessive response to a noxious stimulus), and hyperesthesia (an excessive response to a contact stimulus), though it is not limited to these. As the diseases with neuropathic pain, pain accompanying diabetic neuropathy, cancer pain, trigeminal neuralgia, phantom limb pain, postherpetic neuralgia, fibromyalgia, low back/leg pain, thalamic pain, atypical facial pain such as entrapment (compressive) peripheral neuropathy, pain accompanying spinal cord injury, pain accompanying multiple sclerosis, pain accompanying chemotherapy-induced neuropathy, cancer pain on which an analgesic effect of a narcotic analgesic such as morphine is insufficient, and the like can be exemplified. Further, the injured nerve may be either a central or peripheral nerve, and the type of neuropathy may be either mononeuropathy or polyneuropathy.

The compound of the formula (I) and/or a pharmaceutically acceptable salt thereof can be easily obtained by the production method described in Patent document 1 and Non-patent document 4 or a production method based on those methods.

The compound of the formula (I) may have one or more asymmetric centers, and in that case, it may be present as an enantiomer or a diastereomer. In the invention, a mixture of these isomers and the respective isomers separated from each other are all included.

Accordingly, for example, in addition to (±)-2-[(inden-7-yloxy)methyl]morpholine, its enantiomers, (+)-2-[(inden-7-yloxy)methyl]morpholine and (-)-2-[(inden-7-yloxy)methyl]morpholine are included in the compound of the formula (I).

The compound of the formula (I) can be formed into salts with various acids by a common procedure. The salt of the compound (I) is a pharmaceutically acceptable salt, and examples thereof include organic acid salts (such as acetates, malonates, tartrates, methanesulfonates, benzenesulfonates, formates, toluenesulfonates, and trifluoroacetates), inorganic acid salts (such as hydrochlorides, hydrobromides, sulfates, and phosphates), and amino acid salts (such as alginates, aspartates, and glutamates). Accordingly, the invention includes all morpholine derivatives represented by the formula (I) and pharmaceutically acceptable salts thereof.
The compound of the formula (I) can form hydrates and various pharmaceutically acceptable solvates. These hydrates and solvates are also included in the invention.

A pharmaceutical preparation of the invention can be prepared by a commonly used procedure using a pharmaceutical carrier, excipient, and the like which are commonly used in this field. The administration may be either oral administration by a tablet, a pill, a capsule, a granule, a powder, a liquid, or the like, or parenteral administration by an injection (intraarticular, intravenous, intramuscular, or the like), a suppository, an eye drop, an eye ointment, a transdermal liquid, an ointment, a transdermal adhesive patch, a transmucosal liquid, a transmucosal adhesive patch, an inhalant, or the like.

As a solid composition for oral administration in the invention, a tablet, a powder, a granule, or the like are used. In such a solid composition, one or more active ingredients are mixed with at least one inert diluent such as lactose, mannitol, glucose, hydroxypropyl cellulose, microcrystalline cellulose, starch, polyvinylpyrrolidone, and/or magnesium metasilicate aluminate. The composition may contain an additive other than the inert diluent, for example, a lubricant such as magnesium stearate, a disintegrating agent such as cellulose calcium glycolate, a stabilizing agent, or a solubilizing agent according to a common procedure. The tablet or pill may be coated with a sugar coating such as sucrose, gelatin, hydroxypropyl cellulose, hydroxypropylmethyl cellulose phthalate, or the like, or a film such as a gastric-soluble or enteric-soluble substance, as needed.

A liquid composition for oral administration includes a pharmaceutically acceptable emulsion, solution, suspension, syrup, elixir, and the like, and contains a commonly used inert diluent such as purified water or ethanol. The liquid composition may further contain an auxiliary agent such as a solubilizing agent, a wetting agent, or a suspending agent, a sweetener, a flavor, a perfume, or a preservative other than the inert diluent.

The injection for parenteral administration contains a sterile aqueous or non-aqueous solution, suspension or emulsion. Examples of the aqueous solution or suspension include distilled water for injection and physiological saline. Examples of the non-aqueous solution or suspension include propylene glycol, polyethylene glycol, vegetable oils such as olive oil, alcohols such as ethanol, and Polysorbate 80 (Pharmacopoeia name). Such a composition may further contain a tonicity agent, a preservative, a wetting agent, an emulsifier, a dispersant, a stabilizing agent, or a solubilizing agent. These are sterilized by, for example, filtration through a bacteria-trapping filter, the addition of a bactericide thereto, or irradiation. Alternatively, a sterile solid composition is prepared, and the resulting composition can be used by being dissolved or suspended in sterile water or a sterile solvent for injection before use.

As a transmucosal preparation such as a transnasal preparation, a solid, a liquid, or a semi-solid preparation are used, and such a preparation can be prepared according to a conventionally known method. For example, a known pH adjusting agent, preservative, thickening agent or excipient are appropriately added and the resulting mixture is formed into a solid, liquid or semi-solid preparation. The transnasal preparation is administered using a common spray apparatus, natal spray container, tube, intranasal insert, or the like.

A medicinal agent to be used in the invention is administered to a patient with neuropathic pain, and a suitable daily dose is, in the case of usual oral administration, from about 0.001 to 100 mg/kg of body weight, preferably from about 0.01 to 100 mg/kg of body weight, more preferably from about 0.01 to 10 mg/kg of body weight. The daily dose is administered once per day or two to four times per day by dividing it into two to four portions. In the case of intravenous administration, a suitable daily dose is from about 0.0001 to 10 mg/kg of body weight, and it is administered once to several times per day by dividing it into one to several portions. Further, in the case of a transmucosal preparation, a dose of about 0.001 to 100 mg/kg of body weight is administered once to several times per day by dividing it into one to several portions. The dose is appropriately determined depending on the individual cases by taking into consideration the symptoms, age, sex, and the like.

### Example

The following Example is for the purpose of illustrating the present invention in further detail and is not intended to limit the invention. The invention is fully illustrated by way of the Example, however, it will be apparent to those skilled in the art that various modifications and variations of the Example can be made in the invention. Accordingly, such modifications and variations are included in the invention as long as they do not depart from the scope of the invention.

### Example 1

### (Experiment)

The evaluation of an analgesic effect on neuropathic pain was performed according to the method of Kim et al. using L5/L6 spinal nerve ligation models. That is, the left L5 and L6 sciatic nerves of male SD rats were tightly ligated with a silk thread under pentobarbital anesthesia, and thereafter, the evaluation was performed. As the assessment of pain behavior, a von Frey Test on the plantar surface of the animals was employed (Pain, vol. 50, pp. 355-363, 1992). That is, the plantar surface of the operated limb of the nerve ligation rat was stimulated by using von Frey Filament until a withdrawal response was observed, and the lowest amount of force (g) required to elicit withdrawal response was defined as a pain threshold. The evaluation of compounds was performed in a period between 7 and 14 days after the operation in which a decrease in the threshold was stably observed. On the previous day of the evaluation of compounds, a von Frey Test was performed and the rats were grouped so as to minimize the variation in the mean value of pain threshold. Each of a vehicle and diluted solutions obtained by diluting (±)-2-[(inden-7-yloxy)methyl]morpholine hydrochloride (indeloxazine hydrochloride), (+)-2-[(inden-7-yloxy)methyl]morpholine hydrochloride ((+)-indeloxazine hydrochloride), (-)-2-[(inden-7-yloxy)methyl]morpholine hydrochloride ((-)-indeloxazine hydrochloride), or duloxetine with a vehicle so as to give a dose of 30 mg/kg was orally administered to rats in each group. The oral absorption of venlafaxine is poor, therefore, venlafaxine was intraperitoneally administered. As the vehicle, distilled water was used. A pain threshold was measured at 3 hours after administration in the case of duloxetine, and at 1 hour after administration in the case of the other compounds. An analgesic effect of each compound was examined based on recovery of the pain threshold in the each compound administration group compared with that in the vehicle administration group, as S.K. Joshi et al. described (Neuroscience, vol. 143, pp. 587-596, 2006). Statistical test was performed between the vehicle administration group and each of the drug administration groups using a Student's t-test.

### (Results)

The results of the von Frey Test are shown in Table 1. The numerical values in the table indicate a mean value ± standard error of the mean (SEM) of recovery ratios of thresholds of compounds when the pain threshold of the normal side paw is regarded as 100% and the pain threshold of the operated side paw is regarded as 0%. Each (±)-2-[(inden-7-yloxy)methyl]morpholine hydrochloride, (+)-2-[(inden-7-yloxy)methyl]morpholine hydrochloride, and (-)-2-[(inden-7-yloxy)methyl]morpholine hydrochloride administration group at an oral dose of 30 mg/kg caused significant recovery in the pain threshold compared with the vehicle group. Furthermore, these compounds recovered the pain threshold in operated side of paw as the same level as that in the normal paw. These results indicate that these three compounds had an analgesic efficacy to restore the drop in pain threshold relevant to neuropathic symptom (abnormal paresthesia such as allodynia) to a normal level. On the other hand, in the cases of the administration of duloxetine or venlafaxine at a dose of 30 mg/kg, a significant recovery in the threshold was not observed between these compound administration groups and the vehicle group, and it was a partial effect.

**[Table 1]**

| Compound | % Effect (30 mg/kg, p.o.) |
|---|---|
| (±)-Indeloxazine hydrochloride | 94.3±24.6 ** |
| (+)-Indeloxazine hydrochloride | 94.5±17.0 *** |
| (-)-Indeloxazine hydrochloride | 115.7±22.0 *** |
| Duloxetine | 49.3+25.3 |
| Venlafaxine | 37.1±23.1 |

The symbol "***" in the table indicates that as a result of the Student's t-test, there is a significant difference compared with the vehicle group at a significance level less than 0.5%.
The symbol "**" in the table indicates that as a result of the Student's t-test, there is a significant difference compared with the vehicle group at a significance level less than 1%.

### Industrial Applicability

A pharmaceutical composition of the invention is useful for providing an excellent preventive and/or therapeutic agent for neuropathic pain, and is particularly useful for providing a preventive and/or therapeutic agent for allodynia, hyperalgesia, hyperesthesia, spontaneous pain, cancer pain, trigeminal neuralgia, phantom limb pain, postherpetic neuralgia, fibromyalgia, low back/leg pain, thalamic pain, entrapment (compressive) peripheral neuropathy, atypical facial pain, pain accompanying spinal cord injury, pain accompanying multiple sclerosis, pain accompanying chemotherapy-induced neuropathy, cancer pain on which an analgesic effect of a narcotic analgesic such as morphine is insufficient, pain accompanying diabetic neuropathy, and the like.

## Claims

1. An agent for preventing and/or treating neuropathic pain comprising a compound of the formula (I): 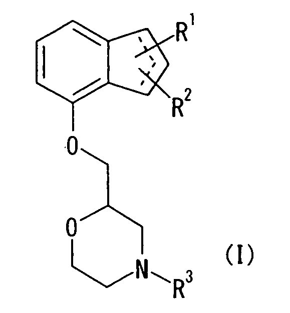 (wherein R¹ and R² are the same or different and represent a hydrogen atom, a lower alkyl , or a phenyl ;
R³ represents hydrogen, a lower alkyl , a phenyl , or a benzyl ; and
a dotted line indicates that a double bond can be formed) or a pharmaceutically acceptable salt thereof as an active ingredient.

2. The agent according to claim 1, wherein the compound of the formula (I) or a pharmaceutically acceptable salt thereof is (±)-2-[(inden-7-yloxy)methyl]morpholine or a pharmaceutically acceptable salt thereof.

3. The agent according to claim 1, wherein the compound of the formula (I) or a pharmaceutically acceptable salt thereof is (+)-2-[(inden-7-yloxy)methyl]morpholine or a pharmaceutically acceptable salt thereof.

4. The agent according to claim 1, wherein the compound of the formula (I) or a pharmaceutically acceptable salt thereof is (-)-2-[(inden-7-yloxy)methyl]morpholine or a pharmaceutically acceptable salt thereof.

5. Use of a compound of the formula (I): 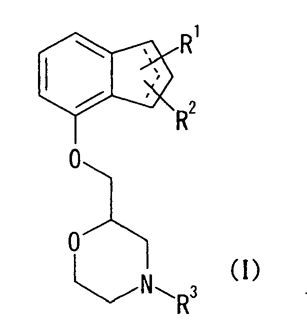 (wherein R¹ and R² are the same or different and represent a hydrogen atom, a lower alkyl , or a phenyl ;
R³ represents hydrogen, a lower alkyl , a phenyl , or a benzyl ; and
a dotted line indicates that a double bond can be formed) or a pharmaceutically acceptable salt thereof for manufacture of a medicament for preventing and/or treating neuropathic pain.

6. The use according to claim 5, wherein the compound of the formula (I) or a pharmaceutically acceptable salt thereof is (±)-2-[(inden-7-yloxy)methyl]morpholine or a pharmaceutically acceptable salt thereof.

7. The use according to claim 5, wherein the compound of the formula (I) or a pharmaceutically acceptable salt thereof is (+)-2-[(inden-7-yloxy)methyl]morpholine or a pharmaceutically acceptable salt thereof.

8. The use according to claim 5, wherein the compound of the formula (I) or a pharmaceutically acceptable salt thereof is (-)-2-[(inden-7-yloxy)methyl]morpholine or a pharmaceutically acceptable salt thereof.

9. A method for preventing and/or treating neuropathic pain comprising administering an effective amount of a compound of the formula (I): 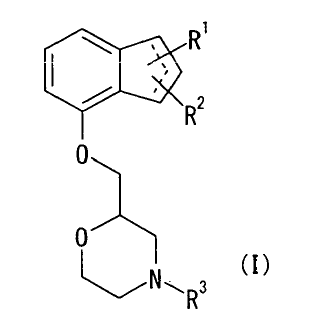 (wherein R¹ and R² are the same or different and represent a hydrogen atom, a lower alkyl , or a phenyl ;
R³ represents hydrogen, a lower alkyl , a phenyl , or a benzyl ; and
a dotted line indicates that a double bond can be formed) or a pharmaceutically acceptable salt thereof.

10. The method according to claim 9, wherein the compound of the formula (I) or a pharmaceutically acceptable salt thereof is (±)-2-[(inden-7-yloxy)methyl]morpholine or a pharmaceutically acceptable salt thereof.

11. The method according to claim 9, wherein the compound of the formula (I) or a pharmaceutically acceptable salt thereof is (+)-2-[(inden-7-yloxy)methyl]morpholine or a pharmaceutically acceptable salt thereof.

12. The method according to claim 9, wherein the compound of the formula (I) or a pharmaceutically acceptable salt thereof is (-)-2-[(inden-7-yloxy)methyl]morpholine or a pharmaceutically acceptable salt thereof.
